# EUROPÄISCHE PATENTANMELDUNG

(11) **EP 1 249 248 A1**
(43) Veröffentlichungstag der Anmeldung: **16.10.2002**
(21) Anmeldenummer: 01101334.9
(22) Anmeldetag: 12.04.2001
(51) Int. Cl.: A61L 9/12, B60H 3/00

(54) **Duftstoffspender mit parfümierten Lüftungsplättchen**

(71) Anmelder: Zimmermann, Steffen, 04821 BRandis (DE)
(72) Erfinder: Zimmermann, Steffen, 04821 BRandis (DE)

(57) **Zusammenfassung**

Die Erfindung betrifft einen Duftstoffspender, der einen Aufnahmebehälter aus Kunststoff mit parfümierten Filzstreifen aufweist. Der Aufnahmebehälter ist so konstruiert, dass er durch Aufsteckelemente auf die Lüftungsklappen eines PKWs aufgesteckt werden kann.

## Beschreibung

Der AFRO-FIX 2000 dient zur Erfrischung der PKW-Kabinenluft bei eingestellter Ventilation.

Bei dem angemeldeten Gegenstand handelt es sich um einen nachfüllbaren Kunststoffbehälter für die Aufnahme von parfümierten Lüftungsblättchen (Filzstreifen. Gleichzeitig dient die Frontfläche zur Aufnahme von bedruckten Werbeaufklebern.

Der vorgenannte Aufnahmebehälter besteht aus verschiedenfarbigem Kunststoff (PE) in den durch eine seitliche Öffnung ein fester parfümierter Filzsfreifen (Lüftungsblättchen) eingeführt wird.
Bei der Vermarktung des AERO-FIX 2000 wird der Kunststoffaufnahmebehälter erstmals zusammen mit einem bereits enthaltenen parfümierten Lüftungsblättchen käuflich erworben.

Der Kunststoffaufnahmebehälter wurde so konstruiert, das er durch seine einseitigen Aufsteckelemente auf die Lüftungsklappen eines beliebigen Personenkraftwagen (PKW) einfach aufgesteckt werden kann. Wird im PKW die Zufuhr von Frisch- bzw. erwärmter Umluft durch Zuschalten der Ventilation eingestellt, gelangt die ventilierte Luft durch diverse Lüftungsöffnungen bis in den Innenbereich des Kunststoffaufnahmebehälter und umspült dort den parfümierten Filzstreifen. Bei der Umspülung des Filzstreifen wird die Luft wiederum mit freiwerdenden Parfümpartikeln angereichert und gelangt durch die vorbeschriebenen Lüftungsöffnungen im Kunststoffaufnahmebehälter so in das Innere der PKW-Kabine. Hier sorgt die angereicherte Luft für einen angenehm frischen Wohlgeruch.

Der nachfüllbare Kunststoffaufnahmebehälter wurde so konstruiert, das das parfümierte Lüftungsblättchen (Filzstreifen) nach Verbrauch des Parfüms einfach aus dem Kunststoffaufnahmebehälter entfernt und durch ein neu erworbenes frisch parfümiertes Lüftungsblättchen wieder ersetzt werden kann.
Beim Nachfüllen eines neuen parfümierten Lüftungsblättchen muß lediglich das verbrauchte Lüftungsblättchen entsorgt werden. Der Kunststoffaufnahmebehälter wird hingegen aus ökologischen Gründen weiter verwendet.

Bei der Sichtfläche des Kunststoffaufnahmebehälter handelt es sich um eine geschlossene Oberfläche, die bei der Herstellung des Kunststoffaufnahmebehälter flächendeckend mit verschiedenen bedruckten Werbeaufklebern versehen wird.

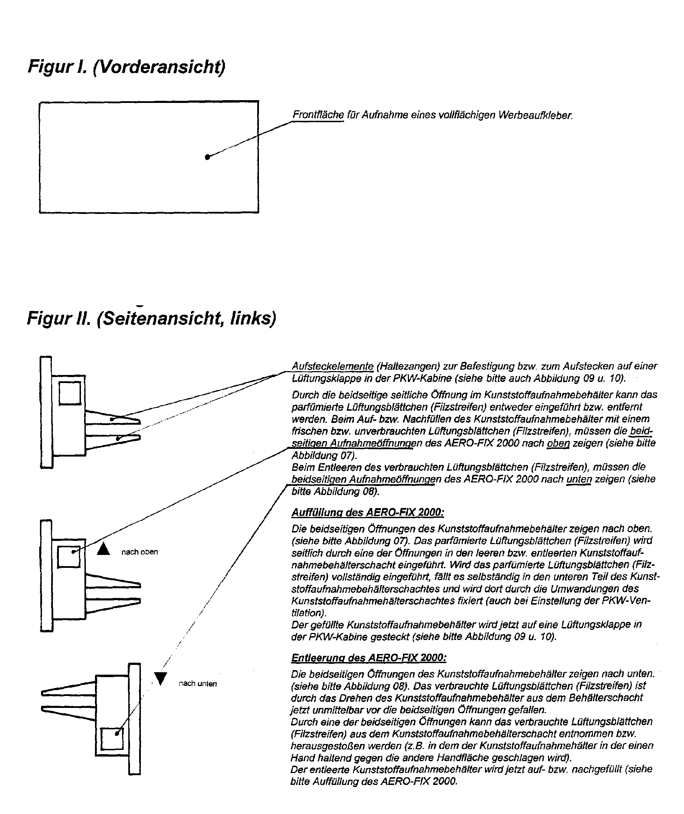

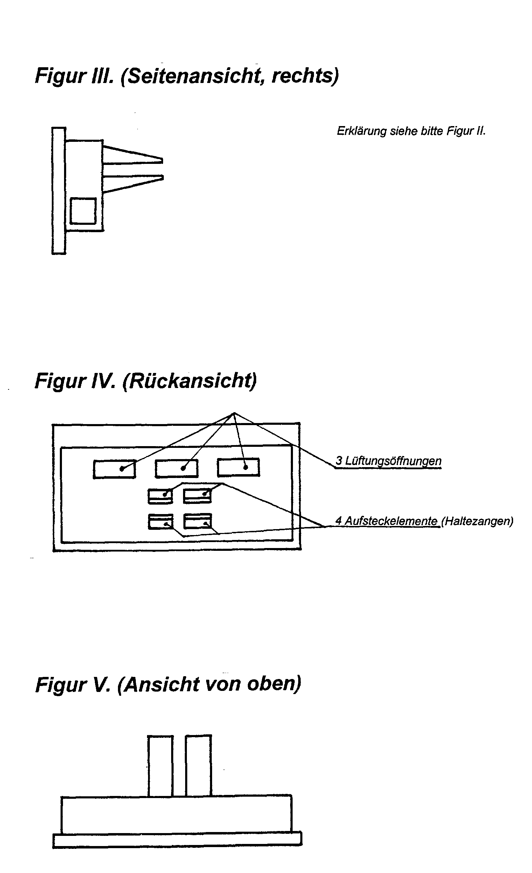

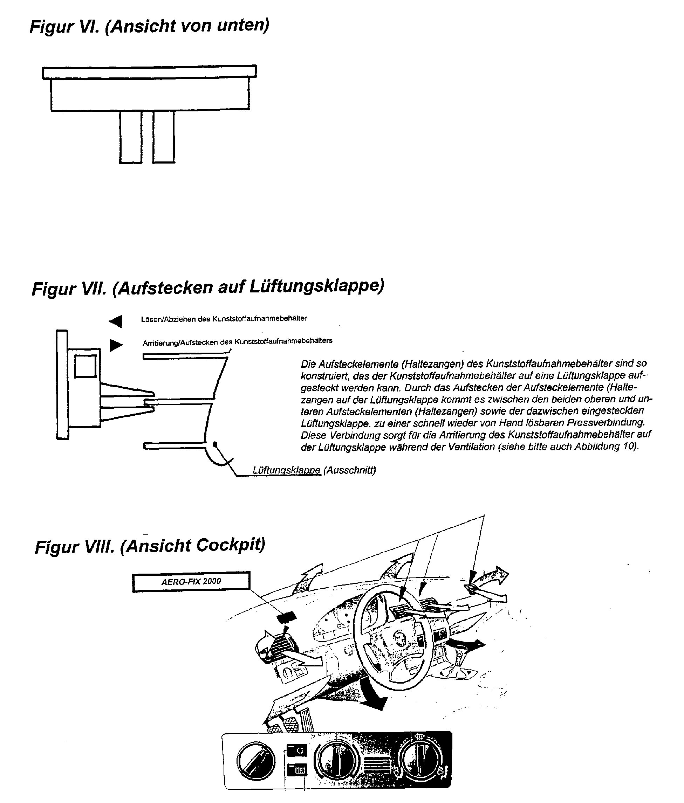

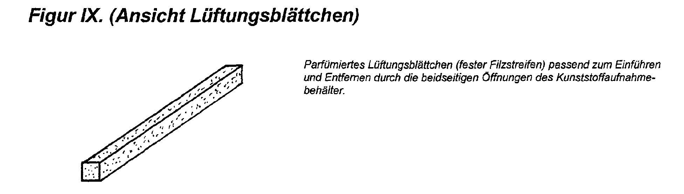

## Patentansprüche

1. Bei dem angemeldeten Gegenstand handelt es sich um einen nachfüllbaren Kunststoffbehälter für die Aufnahme von parfümierten Lüftungsblättchen (Filzstreifen) und einer Frontfläche für die Aufnahme eines bedruckten Werbeaufklebers.
Der vorgenannte Aufnahmebehälter besteht aus Kunststoff (PE) in den durch eine seitliche Öffnung ein fester parfümierter Filzstreifen (Lüftungsblättchen) eingeführt wird.
Bei der Vermarktung des AERO-FIX 2000 wird der Kunststoffaufnahmebehälter erstmals zusammen mit einem bereits enthaltenen parfümierten Lüftungsblättchen käuflich erworben.
Der Kunststoffaufnahmebehälter wurde so konstruiert, das er durch seine einseitigen Aufsteckelemente auf die Lüftungsklappen eines beliebigen Personenkraftwagen (PKW) einfach aufgesteckt werden kann. Wird im PKW die Zufuhr von Frisch- bzw. erwärmter Umluft durch Zuschalten der Ventilation eingestellt, gelangt die ventilierte Luft durch diverse Lüftungsöffnungen bis in den Innenbereich des Kunststoffauf nahmebehälter und umspült dort den parfümierten Filzstreifen. Bei der Umspülung des Filzstreifen wird die Luft wiederum mit freiwerdenden Parfüm partikeln angereichert und gelangt durch die vorbeschriebenen Lüftungsöffnungen im Kunststoffaufnahmebehälter so in das Innere der PKW-Kabine. Hier sorgt die angereicherte Luft für einen angenehm frischen Wohlgeruch.
Der nachfüllbare Kunststoffaufnahmebehälter wurde so konstruiert, das das parfümierte Lüftungsbläffchen (Filzstreifen) nach Verbrauch des Parfüms einfach aus dem Kunststoffaufnahmebehälter entfernt und durch ein neu erworbenes frisch parfümiertes Lüftungsblättchen wieder ersetzt werden kann.
Beim Nachfüllen eines neuen parfümierten Lüftungsblättchen muß lediglich das verbrauchte Lüftungsblättchen entsorgt werden. Der Kunststoffaufnahmebehälter wird hingegen aus ökologischen Gründen weiter verwendet.
Bei der Sichtfläche des Kunststoffaufnahmebehälter handelt es sich um eine geschlossene Oberfläche, die bei der Herstellung des Kunststoffaufnahmebehälter flächendeckend mit verschiedenen bedruckten Werbeaufklebern versehen wird.
Für den vorbeschriebenen Gegenstand besteht meinerseits ein Patentanspruch.
